(19) Europäisches Patentamt
European Patent Office
Office européen des brevets

(11) EP 4 803 074 A1

(12) EUROPEAN PATENT APPLICATION
published in accordance with Art. 153(4) EPC

(43) Date of publication:
**09.09.2026 Bulletin 2026/37**

(21) Application number: **24884924.2**

(22) Date of filing: **31.10.2024**

(51) International Patent Classification (IPC):
***A61K 9/32*** *(2006.01)* ***A61K 31/506*** *(2006.01)*
***A61K 9/20*** *(2006.01)* ***A61K 47/26*** *(2006.01)*
***A61K 47/38*** *(2006.01)* ***A61P 35/00*** *(2006.01)*

(52) Cooperative Patent Classification (CPC):
**A61K 9/20; A61K 9/28; A61K 31/506; A61K 47/26; A61K 47/38; A61P 11/00; A61P 35/00**

(86) International application number:
**PCT/CN2024/129036**

(87) International publication number:
**WO 2025/092926 (08.05.2025 Gazette 2025/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.10.2023 CN 202311442387**

(71) Applicant: **TYK Medicines, Inc.**
**Huzhou, Zhejiang 313100 (CN)**

(72) Inventors:
- **NIU, Chengshan**
  **Zhengzhou, Henan 451162 (CN)**
- **ZHENG, Maolin**
  **Zhengzhou, Henan 451162 (CN)**
- **WANG, Guohui**
  **Zhengzhou, Henan 451162 (CN)**
- **LIANG, Apeng**
  **Huzhou, Zhejiang 313100 (CN)**
- **CHEN, Shaoqing**
  **Huzhou, Zhejiang 313100 (CN)**
- **LI, Jun**
  **Huzhou, Zhejiang 313100 (CN)**
- **WU, Yusheng**
  **Huzhou, Zhejiang 313100 (CN)**
- **LI, Xue**
  **Huzhou, Zhejiang 313100 (CN)**
- **QIU, Jingwen**
  **Huzhou, Zhejiang 313100 (CN)**
- **ZHEN, Zirui**
  **Huzhou, Zhejiang 313100 (CN)**

(74) Representative: **dompatent Partnerschaft von Patentanwälten und Rechtsanwälten mbB
Deichmannhaus am Dom
Bahnhofsvorplatz 1
50667 Köln (DE)**

# (54) PHARMACEUTICAL COMPOSITION OF PYRIMIDINE DERIVATIVE

(57) Disclosed are a pharmaceutical composition of N-(2-((2-(dimethylamino)ethyl)(methyl)amino)-4-methoxy-5-((4-(1-deuteromethyl-1H-indol-3-yl)pyrimidin-2-yl)amino)phenyl)acrylamide methanesulfonate (TY-9591), a method for preparing same, and use thereof. The pharmaceutical composition is a coated tablet, with a tablet core comprising TY-9591, mannitol, microcrystalline cellulose, low-substituted hydroxypropyl cellulose, and sodium stearyl fumarate. By optimizing the formula and the preparation process, the composition exhibits excellent storage stability, accelerated stability, and dissolution performance.

EP 4 803 074 A1

## Description

### TECHNICAL FIELD

The present invention relates to the field of medicine, specifically to a pharmaceutical composition comprising a pyrimidine derivative.

### BACKGROUND ART

The chemical name of the free base TY-9591 is N-(2-((2-(dimethylamino)ethyl) (methyl)amine)-4-methoxy-5-((4-(1-deuteriated methyl-1H-indol-3-yl)pyrimidin-2-yl)amine)phenyl)acrylamide, and its structural formula is shown in formula I.

Formula I

Pharmaceutically acceptable salts of the compound shown in Formula I are hereinafter referred to as "the drug."
TY-9591 is the mono-methanesulfonate salt of the compound shown in Formula I.
TY-9591 may potentially be used for the treatment of diseases or conditions in which EGFR or other protein kinases are inhibited by TY-9591. TY-9591 is disclosed in CN104140418B, which is incorporated herein by reference. Example 3 of CN104140418B describes the synthesis of TY-9591 and notes that the product was obtained in solid form. CN110950847B describes several free base polymorphic forms of TY-9591, and the patent is incorporated herein by reference. CN108558835A describes the crystal forms of TY-9591, and the patent is incorporated herein by reference.

Formula II

Chinese Patent 201280033773.9 discloses the compound AZD9291 (Formula II), which is effective against tumors harboring the T790M EGFR mutation. However, it is readily demethylated in vivo, increasing the metabolic burden on the liver and causing hepatotoxicity. This leads to increased toxicity of the metabolites and results in a short in vivo half-life, ultimately affecting the drug's anticancer activity. In contrast, TY-9591 can reduce the occurrence of this side effect. To deliver the therapeutic benefits of TY-9591 to patients in need, it is necessary to formulate TY-9591 into a pharmaceutical composition, particularly a solid dosage form suitable for oral administration. Therefore, there is a need for a TY-9591 pharmaceutical formulation that is of stable quality, with good in vivo dissolution, high bioavailability, and good storage and accelerated stability.
Chinese Patent 201810017685.9 discloses a pharmaceutical formulation of an AZD9291 deuterated derivative in the form of an immediate-release tablet; however, the patent specifies that the D90 of the active ingredient must be 20 - 280 μm during tablet preparation. This is primarily because the tablet manufacturing process cannot ensure the stability of small particle sizes (<20 μm). When the active ingredient particles become smaller, their specific surface area increases, which can easily lead to decomposition, deliquescence, and other issues, resulting in reduced dissolution rates and instability of the resulting pharmaceutical formulation. When D90 exceeds 280 μm, due to the inherent poor solubility of the AZD9291 deuterated derivative, excessively large drug particles result in low dissolution rates for the formulated drug (e.g., in Example 1(e), when the API particle size was D90 = 320 μm, the dissolution rate of the resulting tablet was only 90% within 60 minutes). This lack of control over particle size hinders the scale-up of the formulation.
Based on this, the present invention overcomes particle size limitations, enabling the preparation of formulations with good stability and rapid dissolution within the range of active ingredient particle sizes smaller than 20 μm and larger

than 280 μm.

Patent CN105848647A discloses a pharmaceutical composition of AZD9291 in the form of an immediate-release tablet containing AZD9291 or a salt thereof; however, this patent is specifically designed for AZD9291. Furthermore, the patent employs a lengthy mixing process (e.g., in Example 8 of CN105848647A, the total mixing time for three mixing steps reaches 73 minutes). In scaled-up production of the formulation, prolonged mixing hinders the control of drug stability, limits production capacity, and results in higher production costs. Based on this patent, the present invention optimizes the preparation process parameters. The mixing speeds for all three mixing steps are lower than those in the patent, while the total mixing time is reduced to one-third of that in the patent. This significantly shortens the formulation production cycle, reduces production costs, and facilitates the protection of drug stability. Furthermore, the present invention employs a lower coating weight gain (3 - 4% vs. 5% in Example 9 of Patent CN105848647A for the same dosage form of 40 mg), which similarly contributes to improved drug stability. At the same time, the formulation prepared using the process of the present invention exhibits a faster dissolution rate, particularly during the early stages of drug dissolution (5 min and 10 min); compared to Example 9 of Patent CN105848647A, the drug dissolution rate is significantly improved.

## SUMMARY OF THE INVENTION

The objective of the present invention is to provide a TY-9591 drug formulation that exhibits good in vivo dissolution, high bioavailability, and excellent storage stability (under both long-term and accelerated conditions), as well as a method preparation therefor and its use in the treatment of tumors.

In a first aspect of the present invention, provided is a pharmaceutical composition, wherein the pharmaceutical composition is a tablet comprising a tablet core and a coating located on the outer surface of the tablet core;

the tablet core comprises the following components:

| | Part by weight | Function |
|---|---|---|
| Methanesulfonate salt of Compound of Formula I | 18.5 - 19.5 (preferably 19-19.1, the most preferably 19.04) | Active ingredient |
| Mannitol | 57 - 60 (preferably 58-59, the most preferably 58.96) | Primary diluent |
| Microcrystalline cellulose | 12 - 17 (preferably 14-16, the most preferably 15) | Secondary diluent |
| Low-substituted hydroxypropyl cellulose | 3-7 (preferably 4-6, the most preferably 5) | Disintegrant |
| Sodium stearoyl fumarate (internal) | 0.3 - 0.7 (preferably 0.4-0.6, the most preferably 0.5) | Primary lubricant |
| Sodium stearoyl fumarate (external) | 1.3 - 1.7 (preferably 1.4-1.6, the most preferably 1.5) | Secondary lubricant |

the coating completely covers the tablet core, and the mass of the coating is 1 - 6% of the mass of the tablet core (preferably 2.5-5wt%, the most preferably 2-4wt%);

Formula I

the pharmaceutical composition was prepared as follows:

1) providing the methanesulfonate salt of the compound of Formula I, mannitol, microcrystalline cellulose, low-substituted hydroxypropyl cellulose, sodium stearoyl fumarate, and a coating suspension;
2) mixing the methanesulfonate salt of the compound of Formula I, mannitol, microcrystalline cellulose, and low-substituted hydroxypropyl cellulose to obtain a first mixture;
3) mixing the sodium stearoyl fumarate for internal incorporation with the first mixture to obtain a second mixture;

4) subjecting the second mixture to dry granulation to obtain a first granules;
5) mixing the first granules with sodium stearoyl fumarate for external incorporation to obtain the third mixture;
6) pressing the third mixture to obtain a plain tablet;
7) coating the plain tablet with the coating suspension to obtain the pharmaceutical composition.

In another preferred embodiment, the D90 of the active ingredient is 1 - 500 μm (preferably 3-495μm).

In another preferred embodiment, the methanesulfonate salt of the compound of formula I is the mono-methanesulfonate crystal form of the compound of formula I.

In another preferred embodiment, the X-ray powder diffraction patterns of the crystal form having diffraction peaks at 2θ angle (where the unit of the 2θ angle is °): 7.1±0.2, 8.5±0.2, 9.4±0.2, 10.3±0.2, 12.6±0.2, 14.4±0.2, 15.1 ±0.2, 15.6±0.2, 16.3±0.2, 17.0±0.2, 17.3±0.2, 17.7±0.2, 18.2±0.2, 18.7±0.2, 19.4±0.2, 19.7±0.2, 20.2±0.2, 20.7 ±0.2, 21.6±0.2, 22.0±0.2, 22.8±0.2, 23.5±0.2, 24.2±0.2, 24.8±0.2, 25.6±0.2, 26.0±0.2, 26.9±0.2, 27.7±0.2, 28.2 ±0.2, 29.5±0.2, 30.7±0.2, 31.7±0.2, 32.5±0.2, 33.1±0.2, 33.8±0.2, 34.6±0.2, 34.9±0.2, 35.6±0.2, 37.9±0.2, and 38.7±0.2.

In another preferred embodiment, the coating comprises a stabilizing agent and a polymer.

In another preferred embodiment, the stabilizing agent is selected from the group consisting of titanium dioxide, talc, red iron oxide, or a combination thereof.

In another preferred embodiment, the polymer is selected from the group consisting of: polyvinyl alcohol, polyethylene glycol, or a combination thereof.

In another preferred embodiment, the pharmaceutical composition has one or more of the following features:

1) In a sodium chloride buffer solution at pH 1.2, the dissolution rate of the active ingredient in the pharmaceutical composition is ≥50% (preferably ≥70%, the most preferably ≥90%) at 10 minutes;
2) In a sodium chloride buffer solution at pH 1.2, the dissolution rate of the active ingredient in the pharmaceutical composition is ≥80% (preferably ≥90%, the most preferably ≥93%) at 15 minutes;
3) In a sodium chloride buffer solution at pH 1.2, the dissolution rate of the active ingredient in the pharmaceutical composition is ≥85% (preferably ≥90%, the most preferably ≥94%) at 20 minutes;
4) In a sodium chloride buffer solution at pH 1.2, the dissolution rate of the active ingredient in the pharmaceutical composition is ≥90% (preferably ≥92%, the most preferably ≥95%) at 30 minutes;
5) In a sodium chloride buffer solution at pH 1.2, the dissolution rate of the active ingredient in the pharmaceutical composition is ≥96% (preferably ≥97%) at 45 minutes;
6) In a sodium chloride buffer solution at pH 1.2, the dissolution rate of the active ingredient in the pharmaceutical composition is ≥100% at 60 minutes;
7) After storage of the pharmaceutical composition for 3 years at 25±2°C and 60%±5% RH, the dissolution rate of the active ingredient in the pharmaceutical composition is ≥95% at 30 minutes in a sodium chloride buffer solution at pH 1.3;
8) After storage of the pharmaceutical composition for 6 months at 40±2°C and 75%±5% RH, the dissolution rate of the active ingredient in the pharmaceutical composition is ≥95% at 30 minutes in a sodium chloride buffer solution at pH 1.3.

In another preferred embodiment, the pharmaceutical composition is an oral formulation.

In a second aspect of the present invention, provided is a method for preparing the pharmaceutical composition according to the first aspect of the present invention, it comprises steps of:

1) providing the methanesulfonate salt of the compound of Formula I, mannitol, microcrystalline cellulose, low-substituted hydroxypropyl cellulose, sodium stearoyl fumarate, and a coating suspension;
2) mixing the methanesulfonate salt of the compound of Formula I, mannitol, microcrystalline cellulose, and low-substituted hydroxypropyl cellulose to obtain a first mixture;
3) mixing the sodium stearoyl fumarate for internal incorporation with the first mixture to obtain a second mixture;
4) subjecting the second mixture to dry granulation to obtain a first granules;
5) mixing the first granules with sodium stearoyl fumarate for external incorporation to obtain a third mixture;
6) Pressing the third mixture to obtain a plain tablet;
7) Coating the plain tablet with the coating suspension to obtain the pharmaceutical composition.

In another preferred embodiment, the method comprises one or more of the following features:

1) In step 2), mixing speed of the mixing is 4 - 14 rpm (preferably 8-12 rpm);
2) In step 2), mixing time of the mixing is 10 - 20 min (preferably 14-16 min);

3) In step 3), mixing speed of the mixing is 4 - 14 rpm (preferably 8-12 rpm);
4) in step 3), mixing time of the mixing is 2 - 9 min (preferably 4-6 min);
5) in step 4), roller gap of the dry granulation is 1.5 - 2.5 mm;
6) in step 4), roller speed of the dry granulation is 7.0 - 10.0 rpm;
7) In step 4), hydraulic pressure of the dry granulation is 35 - 50 bar;
8) In step 5), mixing speed of the mixing is 4 - 14 rpm (preferably 8-12 rpm);
9) In step 5), mixing time of the mixing is 2 - 10 min (preferably 3-7 min).

In another preferred embodiment, the hardness of the plain tablet is 70 - 120 N (preferably 95 - 105 N) In a third aspect of the present invention, provided is a use of the pharmaceutical composition according to the first aspect of the present invention in the preparation of an antitumor drug.

In another preferred embodiment, the tumor is lung cancer.

In another preferred embodiment, the lung cancer is non-small cell lung cancer.

It should be understood that in the present invention, any of the technical features specifically described above and below (such as in the Examples) can be combined with each other, so as to constitute new or preferred technical solutions. Due to space limitations, it will not redundantly be described one by one herein.

## DESCRIPTION OF FIGURES

Figure 1 shows the dissolution curves of the tablets obtained in Examples 1 - 6.
Figure 2 shows the dissolution curves of the tablets obtained in Examples 7 to 10.
Figure 3 shows the dissolution curves of the tablets obtained in Examples 11 to 13.
Figure 4 shows the dissolution curves of the tablets obtained in Examples 3, 14, and 15.
Figure 5 shows the dissolution curves of the tablets obtained in Examples 16 to 19.
Figure 6 shows the dissolution curves of the tablets obtained in Examples 3 and 21.
Figures 7 and 8 show the content and dissolution curves from the long-term stability testing of the tablets obtained in Example 22 (the dissolution medium is a pH 1.3 sodium chloride-buffered hydrochloric acid solution, and the dissolution time is 30 min).
Figures 9 and 10 show the content and dissolution curves from the accelerated stability testing of the tablets obtained in Example 22 (the dissolution medium is a pH 1.3 sodium chloride-buffered hydrochloric acid solution, and the dissolution time is 30 min).
Figure 11 shows the dissolution curves of the tablets obtained in Example 3 and Comparative Examples 1 and 2. The dissolution medium is a pH 1.3 sodium chloride-hydrochloric acid buffer solution consisting of 0.2% NaCl and 4 mL of hydrochloric acid.

## DETAILED DESCRIPTION OF THE INVENTION

After long-term and in-depth research, through optimization of the formulation and/or manufacturing process the present inventors obtained a pharmaceutical composition with excellent dissolution properties, pharmacokinetic properties, and storage stability . On above basis, the inventors have completed the present invention.

As used herein, the terms "comprising" or "including (containing)" may be open-ended, semi-closed, or closed. In other words, these terms also include "consisting essentially of" or "consisting of."

Drugs can be absorbed at many different sites along the gastrointestinal tract (including the stomach, duodenum, jejunum, ileum, and colon) following oral administration. Due to the significant pH gradient between the stomach (pH 1 - 3.5) and the small intestine (pH 4 - 8), the pH varies at each absorption site. Our research has shown that TY-9591 exhibits significant pH-dependent solubility and moderate permeability. For example, it has been found that TY-9591 has higher solubility at pH 1 (hydrochloric acid) compared to pH 6.8 (sodium hydroxide-sodium dihydrogen phosphate buffer, 25 mM). In such cases, where the solubility of a drug varies with pH-and specifically when solubility is highest at acidic pH levels-a problem arises: the drug may precipitate out of solution as it passes through the gastrointestinal tract, but the drug should remain in solution to be absorbed, such precipitation may affect the extent and rate of absorption. Compounds with pH-dependent solubility (particularly basic compounds) may exhibit undesirable pharmacokinetic properties, such as poor absorption or low bioavailability, which can lead to significant inter-patient and intra-patient variability.

Therefore, there is a need to identify improved formulations of TY-9591 that exhibit favorable dissolution and pharmacokinetic curves and demonstrate good storage stability. At the same time, we have found that the solid formulations of the present invention exhibit excellent storage stability.

The present invention relates to pharmaceutical compositions suitable for oral administration, and more

specifically relates to pharmaceutical compositions (and pharmaceutical tablets) containing TY-9591.

The present invention provides solutions to one or more of the aforementioned problems and relates to novel pharmaceutical compositions containing the drug. The pharmaceutical compositions of the present invention may be formulated as tablets that exhibit improved dissolution characteristics under physiologically relevant conditions and/or a higher total release of the drug based on physiologically relevant parameters.

Because the compounds of the present invention exhibit excellent antitumor activity, pharmaceutical compositions containing the compounds of the present invention as the main active ingredient may be used to treat, prevent, and alleviate tumor-related diseases.

The pharmaceutical composition of the present invention comprises a safe and effective amount of a compound of the present invention or a pharmacologically acceptable salt thereof, and a pharmacologically acceptable excipient or carrier. In which, "safe and effective amount" is meant that the amount of the compound is sufficient to significantly improve the condition without causing serious side effects. Generally, the pharmaceutical composition contains 1-2000 mg of the compound of the present invention/dose, more preferably, 10-1000 mg of the compound of the present invention/dose. Preferably, the "dose" is a capsule or tablet.

"Pharmaceutically acceptable carrier" means one or more compatible solid or liquid fillers or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween®), wetting agent (such as sodium dodecyl sulfate), coloring agents, flavoring agents, stabilizers, antioxidants, preservatives, pyrogen-free water, etc..

The pharmaceutical composition is an injection, a capsule, a tablet, a pill, a powder, or a granule.

The administration mode of the compound or pharmaceutical composition of the present invention is not particularly limited, and representative administration modes include, but are not limited to, oral, intratumoral, rectal, parenteral (intravenous, intramuscular or subcutaneous) and topical administration.

Solid dosage forms for oral administration include capsules, tablets, pills, powders and granules. In these solid dosage forms, the active ingredient is mixed with at least one conventional inert excipient (or carrier), such as sodium citrate or dicalcium phosphate, or mixed with any of the following components: (a) fillers or compatibilizer, such as starch, lactose, sucrose, glucose, mannitol and silicic acid; (b) binders, such as hydroxymethyl cellulose, alginate, gelatin, polyvinylpyrrolidone, sucrose and arabic gum; (c) humectants, such as glycerol; (d) disintegrating agents such as agar, calcium carbonate, potato starch or tapioca starch, alginic acid, certain composite silicates, and sodium carbonate; (e) retarding solvents, such as wax, (f) absorption accelerators, such as quaternary ammonium compound; (g) wetting agents, such as cetyl alcohol and glyceryl monostearate; (h) adsorbents, such as kaolin; and (i) lubricants, such as talc, calcium stearate, magnesium stearate, solid polyethylene glycol, sodium dodecyl sulfate or mixture thereof. In capsules, tablets and pills, the dosage forms may also contain buffering agents.

Solid dosage forms such as tablets, dragees, capsules, pills and granules can be prepared with coatings and shells such as enteric coatings and other materials known in the art. They may contain opacifying agents and the release of the active compound or compound in such compositions may be released in a portion of the digestive tract in a delayed manner. Examples of embedding components that can be employed are polymeric materials and waxy materials. If necessary, the active compound may also be in microencapsulated form with one or more of the above-mentioned excipients.

Liquid dosage forms for oral administration include pharmaceutically acceptable emulsions, solutions, suspensions, syrups or tinctures. In addition to the active compound, the liquid dosage form may contain inert diluents conventionally used in the art, such as water or other solvents, solubilizers and emulsifiers, such as ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures of these substances.

In addition to these inert diluents, the compositions may contain adjuvants such as wetting agents, emulsifying and suspending agents, sweetening agents, flavoring agents and spices.

In addition to the active compound, the suspension may contain suspending agent, such as ethoxylated isooctadecanol, polyoxyethylene sorbitol and dehydrated sorbitan ester, microcrystalline cellulose, aluminum methoxide and agar, or the mixture thereof etc..

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and nonaqueous carriers, diluents, solvents or excipients include water, ethanol, polyols and any suitable mixtures thereof.

Dosage forms for the compounds of the invention for topical administration include ointments, powders, patches,

sprays and inhalants. The active ingredient is mixed under sterile conditions with a physiologically acceptable carrier and any preservatives, buffers, or propellants which may be required if necessary.

The compounds of the invention can be administered alone or in combination with other pharmaceutically acceptable compounds (e.g., anti-tumor agents).

The treatment method of the present invention can be administered alone or in combination with other treatment means or therapeutic drugs.

When the pharmaceutical composition is used, a safe and effective amount of the compound of the present invention is administered to a mammal(such as a human) in need of treatment, wherein the dosage at the time of administration is the pharmaceutically effective dosage, for people having a body weight of 60kg, the daily dose is usually 1~2000mg, preferably 50~ 1000mg. Of course, specific doses should also consider factors such as the administration route, the health of the patient, etc., which are within the skill of the skilled physician.

The specific embodiments of the present invention describe in detail methods for preparing the pharmaceutical compositions of the present invention; however, these specific methods do not limit the scope of the present invention. The pharmaceutical compositions of the present invention may also be conveniently prepared by optionally combining various preparation methods described herein or known in the art.

Typically, the raw materials and reagents used in the manufacturing process of the pharmaceutical compositions of the present invention are commercially available.

It should be understood that, for film-coating premixes, commercially available and well-known premixes in the art may be used to achieve similar technical effects as those of the present invention.

**Crystal form**

A crystal form refers to the solid state in which a drug exists. Research on drug crystal forms involves studying the fundamental state of the drug. Only by gaining a thorough and comprehensive understanding of the crystal forms of chemical drugs can we identify solid crystal forms that are more suitable for treating diseases. The crystal form of a drug can influence its physicochemical properties, directly affecting the foundation upon which the drug exerts its therapeutic effects in clinical settings. Different crystal forms of the same drug may exhibit significant differences in appearance, solubility, melting point, dissolution rate, and bioavailability, thereby impacting the drug's stability, bioavailability, and therapeutic efficacy. Therefore, identifying the stable crystal form of a compound is of great significance.

The active ingredient of a single drug generally exists in two or more crystalline forms, known as drug crystal forms. Different crystal forms have distinct solubility and dissolution rates, which can affect the drug's clinical therapeutic effects by altering its bioavailability in the body. Differences in drug crystal forms may influence dissolution and absorption in the body, thereby affecting bioavailability, clinical efficacy, and safety. At the same time, the stability of drug crystal forms is also of great importance. To enhance bioavailability, reduce toxicity, and improve therapeutic outcomes, greater emphasis must be placed on the stability of drug crystal forms. A stable crystal form ensures the physicochemical stability of the drug formulation during preparation and storage, maintains good solubility and bioavailability, and guarantees the equivalence of drug batches. Since a single drug often possesses multiple crystal forms, the crystal form that offers superior therapeutic effects and is most clinically suitable is currently referred to as the preferred drug crystal form.

In this invention, the crystal form of TY-9591 is the one described in Patent CN 108558835 A.

Specifically, the crystal form of TY-9591 as described in this invention, as determined by X-ray powder diffraction using Cu-K$\alpha$ radiation, having diffraction peaks at2$\theta$ angle (where the unit of the 2$\theta$ angle is °): 7.1$\pm$0.2, 8.5$\pm$0.2, 9.4$\pm$0.2, 10.3$\pm$0.2, 12.6$\pm$0.2, 14.4$\pm$0.2, 15.1$\pm$0.2, 15.6$\pm$0.2, 16.3$\pm$0.2, 17.0$\pm$0.2, 17.3$\pm$0.2, 17.7$\pm$0.2, 18.2$\pm$0.2, 18.7$\pm$0.2, 19.4$\pm$0.2, 19.7$\pm$0.2, 20.2$\pm$0.2, 20.7$\pm$0.2, 21.6$\pm$0.2, 22.0$\pm$0.2, 22.8$\pm$0.2, 23.5$\pm$0.2, 24.2$\pm$0.2, 24.8$\pm$0.2, 25.6$\pm$0.2, 26.0$\pm$0.2, 26.9$\pm$0.2, 27.7$\pm$0.2, 28.2$\pm$0.2, 29.5$\pm$0.2, 30.7$\pm$0.2, 31.7$\pm$0.2, 32.5$\pm$0.2, 33.1$\pm$0.2, 33.8$\pm$0.2, 34.6$\pm$0.2, 34.9$\pm$0.2, 35.6$\pm$0.2, 37.9$\pm$0.2, and 38.7$\pm$0.2.

More specifically, the crystal form of TY-9591 described in this invention having X-ray powder diffraction data shown in Table A below.

Table A

| NO. | Pos[ ° 2Th.] | d-spacing | Rel. Int. [%] | FWHM [° 2Th.] | Area [cts* ° 2Th.] | Backgr.[cts] | Height [cts] |
|---|---|---|---|---|---|---|---|
| I | 7.1694 | 12.33032 | 22.11 | 0.1299 | 481.33 | 2165.75 | 3756.61 |
| 2 | 8.5659 | 10.32289 | 26.78 | 0.1299 | 582.81 | 1967.15 | 4548.65 |
| 3 | 9.4876 | 9.3221 | 22.75 | 0.1299 | 495.09 | 1836.81 | 3864.03 |
| 4 | 10.3303 | 8.56344 | 60.37 | 0.0974 | 985.48 | 1716.83 | 10255.21 |
| 5 | 12.6663 | 6.98887 | 3.22 | 0.1299 | 70.08 | 1470.38 | 546.96 |

(continued)

| NO. | Pos[ ° 2Th.] | d-spacing | Rel. Int. [%] | FWHM [° 2Th.] | Area [cts* ° 2Th.] | Backgr.[cts] | Height [cts] |
|---|---|---|---|---|---|---|---|
| 6 | 14.476 | 6.11897 | 3.78 | 0.1299 | 82.21 | 1409 | 641.61 |
| 7 | 15.1897 | 5.83306 | 75.69 | 0.1299 | 1647.5 | 1398.91 | 12858.21 |
| 8 | 15.6284 | 5.67026 | 9 | 0.1299 | 195.82 | 1392.61 | 1528.31 |
| 9 | 16.313 | 5.43381 | 54.22 | 0.1299 | 1180.11 | 1382 | 9210.42 |
| 10 | 17.0853 | 5.18989 | 37.2 | 0.1299 | 809.81 | 1371 | 6320.32 |
| 11 | 17.3199 | 5.12012 | 25.44 | 0.0974 | 415.31 | 1367.36 | 4321.85 |
| 12 | 17.7495 | 4.99717 | 26.91 | 0.1299 | 585.64 | 1361.34 | 4570.75 |
| 13 | 18.2399 | 4.86391 | 13.88 | 0.1299 | 302.12 | 1354.48 | 2357.95 |
| 14 | 18.75 | 4.7327 | 45.91 | 0.1299 | 999.19 | 1347 | 7798.41 |
| 15 | 19.4808 | 4.55679 | 80.55 | 0.1299 | 1753.35 | 1336 | 13684.39 |
| 16 | 19.7309 | 4.49959 | 68.71 | 0.0974 | 1121.69 | 1332.3 | 11672.64 |
| 17 | 20.254 | 4.38453 | 34.89 | 0.1299 | 759.49 | 1325 | 5927.61 |
| 18 | 20.7361 | 4.28369 | 58.51 | 0.1299 | 1273.53 | 1317.84 | 9939.53 |
| 19 | 21.6748 | 4.10024 | 24.69 | 0.1624 | 671.89 | 1304 | 4195.09 |
| 20 | 22.0837 | 4.02524 | 49.32 | 0.1624 | 1341.76 | 1298 | 8377.63 |
| 21 | 22.8189 | 3.89719 | 53.53 | 0.1624 | 1456.39 | 1287.72 | 9093.38 |
| 22 | 23.5089 | 3.78434 | 30.48 | 0.1948 | 995.29 | 1277 | 5178.64 |
| 23 | 24.2191 | 3.67495 | 65.23 | 0.1624 | 1774.84 | 1267 | 11081.66 |
| 24 | 24.8508 | 3.58294 | 34.42 | 0.1624 | 936.45 | 1258 | 5846.99 |
| 25 | 25.6538 | 3.47259 | 100 | 0.1948 | 3264.94 | 1245.82 | 16987.88 |
| 26 | 26.0667 | 3.41852 | 72.22 | 0.1624 | 1964.93 | 1240 | 12268.55 |
| 27 | 26.9869 | 3.304 | 8.92 | 0.2922 | 436.74 | 1227 | 1514.94 |
| 28 | 27.725 | 3.2177 | 15.06 | 0.1624 | 409.67 | 1216 | 2557.88 |
| 29 | 28.2775 | 3.15607 | 13.51 | 0.1948 | 441.06 | 1208 | 2294.87 |
| 30 | 29.592 | 3.0188 | 8.39 | 0.1299 | 182.59 | 1188.47 | 1425.02 |
| 31 | 30.762 | 2.9066 | 8.26 | 0.1299 | 179.69 | 1176 | 1402.42 |
| 32 | 31.7251 | 2.82053 | 8.94 | 0.1299 | 194.63 | 1169 | 1519 |
| 33 | 32.5786 | 2.74856 | 10.21 | 0.1948 | 333.42 | 1164 | 1734.8 |
| 34 | 33.1905 | 2.69928 | 767 | 0.3897 | 500.63 | 1159 | 1302.42 |
| 35 | 33.8981 | 2.64453 | 4.01 | 0.1299 | 87.25 | 1155 | 680.98 |
| 36 | 34.6016 | 2.59236 | 4.32 | 0.1624 | 117.6 | 1150 | 734.28 |
| 37 | 34.9163 | 2.56971 | 2.82 | 0.1624 | 76.79 | 1148 | 479.48 |
| 38 | 35.6599 | 2.51781 | 5.14 | 0.1624 | 139.96 | 1143 | 873.91 |
| 39 | 37.9581 | 2.37049 | 4.25 | 0.1624 | 115.72 | 1136 | 722.54 |
| 40 | 38.7101 | 2.32616 | 2.45 | 0.2598 | 106.76 | 1136 | 416.63 |

Compared with the prior art, the present invention has the following main advantages:

(1) The pharmaceutical composition exhibits excellent dissolution properties, pharmacokinetic properties, and storage stability;
(2) The pharmaceutical composition is of consistent quality;

(3) The manufacturing process for the pharmaceutical composition is simple and straightforward, facilitating seamless integration into industrial production;
(4) The pharmaceutical composition is convenient to administer, safe and reliable to use, and well-accepted by patients, offering significant social and economic value.

The present invention will be further illustrated below with reference to the specific examples. It should be understood that these examples are only to illustrate the invention but not to limit the scope of the invention. Experimental methods in which the specific conditions are not specified in the following examples are usually in accordance with conventional conditions such as the conditions described in Sambrook et al., Molecular Cloning: Laboratory Manual (New York: Cold Spring Harbor Laboratory Press, 1989), or in accordance with the conditions recommended by the manufacturer. Unless indicated otherwise, percentage and parts are calculated by weight.
Unless otherwise defined, all professional and scientific terminology used in the text have the same meanings as known to the skilled in the art. In addition, any methods and materials similar or equal with the recorded content can apply to the methods of the invention. The preferred embodiments and the materials described herein are only for demonstration purposes.

**General Raw Materials**

| Material Name | Model |
|---|---|
| crystal form of TY-9591, all drug substances used in the embodiments of the present invention are of this crystal form | N/A |
| Mannitol | 100SD |
| Microcrystalline cellulose | VIVAPUR101 |
| Low-substituted hydroxypropyl cellulose | LH-21 |
| Sodium stearoyl fumarate | PRUV |
| Film-coating premix | 85F94245-CN |

**General Methods**

Dissolution Properties

Dissolution refers to the rate and extent to which a drug is released from solid dosage forms, such as tablets, into a specified solvent. Dissolution is a critical parameter in tablet quality control, and dissolution testing is generally required for poorly soluble drugs. The dissolution test involves placing a specified quantity of a solid dosage form into the basket (or dissolution vessel) of a dissolution apparatus. Under a constant temperature of 37°C ± 0.5°C, the test is conducted at a specified rotation speed and in a specified dissolution medium in accordance with established procedures. Samples are collected at specified intervals, and the amount dissolved is determined.
The dissolution testing conditions are as follows:

Dissolution medium: sodium chloride-hydrochloric acid buffer at pH 1.2 (0.2% NaCl + 7 mL hydrochloric acid) or sodium chloride-hydrochloric acid buffer at pH 1.3 (0.2% NaCl + 4 mL hydrochloric acid)
Volume of dissolution medium: 900 mL
Rotation speed: 50 rpm
Water bath temperature: 37 ± 0.5°C
Sampling time points: 5, 10, 15, 20, 30, 45, 60 - 90 min (maximum speed 150 rpm)
Sampling method: Automatic sampling of 1.5 ml without replenishment, followed by 3.5 ml of rinse solution and 10 ml of washing solution

A pH 1.2 sodium chloride-hydrochloric acid buffer solution allows the solubility of the TY-9591 active ingredient to meet the leaching criteria; Testing of TY-9591 tablets in a pH 1.2 hydrochloric acid-sodium chloride buffer solution showed a parabolic dissolution curve with no inflection points or sudden release peaks. The dissolution rate reached approximately 80% at 15 minutes and exceeded 85% between 45 and 60 minutes.

Determination of Impurity B Content

Impurity B (Impurity RRT1.5, i.e., relative retention time 1.5) is a degradation impurity identified in the stability studies of this formulation and is a specific impurity that requires close monitoring. Systematic studies have revealed that Impurity B (Impurity RRT1.5) is highly unstable under neutral and slightly alkaline conditions, readily degrading into the active pharmaceutical ingredient (API) and other impurities, while remaining relatively stable under slightly acidic conditions. Therefore, the study of Impurity B content is also a key point of the invention.

The method for determining the content of Impurity B is as follows:

1. Chromatographic conditions (HPLC):

| Equipment | High-performance liquid chromatograph equipped with a UV detector |
|---|---|
| chromatograp hy column | Waters Symmetry C18 150*4.6mm, 3.5 μm |
| Mobile phase A | Adjust the pH of a 0.1% aqueous solution of heptafluorobutyric acid to 3.0 using ammonia solution (for example: transfer 1.0 mL of heptafluorobutyric acid to 1000 mL of water, then adjust the pH to 3.0 using ammonia solution) |
| Mobile phase B | Methanol: Acetonitrile = 80:20 (%v/v) |
| Detection wavelength | 254 nm |
| Flow rate: | 1.0 mL/min |
| Injection | 10 μL |
| volume | |
| Column temperature | 40°C |
| Run time: | 55 min |

Gradient table:

| Time (min) | Mobile phase A (%V/V) | Mobile phase B (%V/V) |
|---|---|---|
| 0 | 70 | 30 |
| 40 | 35 | 65 |
| 45 | 20 | 80 |
| 45.1 | 70 | 30 |
| 55 | 70 | 30 |

2. Reagents and Reference Standards

Heptafluorobutyric acid: Chromatographic grade Water: Ultrapure water Ammonia solution: Chromatographic grade or analytical grade
Acetonitrile: Chromatographic grade Methanol: Chromatographic grade
TY-9591 Reference Standard: In-house preparation or commercially purchased
3. Solution Preparation
Dilution solution: methanol: water: heptafluorobutyric acid = 50:50:0.1 (v/v/v)
Blank solution: diluent
Standard solution: Prepare a solution containing 0.5 mg/mL of TY-9591 reference standard (e.g., weigh 50 mg of TY-9591 reference standard, accurately transfer it to a 100-mL volumetric flask, dissolve it with the diluent, dilute to the

mark, and mix thoroughly).

Working test solution: Prepare a solution containing 0.45 mg/mL of TY-9591 (e.g., weigh 45 mg of TY-9591 reference standard, accurately transfer it to a 100 mL volumetric flask, dissolve it with diluent, and make up to mark; mix thoroughly).

Reference solution: Prepare a solution containing 1.0 μg/mL of TY-9591 reference standard (e.g., weigh 50 mg of TY-9591 reference standard, accurately transfer it to a 50 mL volumetric flask, dissolve with the diluent, dilute to the mark, and mix thoroughly; then accurately transfer 5.0 mL of this solution to a 100 mL volumetric flask, dilute to the mark with diluent, and mix thoroughly; then accurately transfer 1.0 mL of this solution to a 50 mL volumetric flask, dilute to the mark with diluent, and mix thoroughly).

Sensitivity solution: Prepare a solution containing 0.5 μg/mL of TY-9591 reference standard (e.g., accurately transfer 5.0 mL of the reference solution to a 10 mL volumetric flask, dilute to the mark with diluent, and mix thoroughly).

Test solution for related substances: Prepare a solution containing 1.0 mg/mL of the test substance (e.g., weigh 50 mg of the test substance, accurately transfer it to a 50 mL volumetric flask, dissolve with diluent, dilute to the mark, and mix thoroughly).

Concentration of the test solution: Prepare a test solution containing 0.5 mg/mL of TY-9591 (e.g., weigh 50 mg of the TY-9591 sample, accurately transfer it to a 100 mL volumetric flask, dissolve with the diluent, and make up to the mark; mix thoroughly).

Prepare two concentration of the test solutions using the same method.

Procedure control solution: Standard solution.

4. Calculation

$$\text{Concentration of impurity B} = \frac{At \times Cr \times P}{Ar \times Ct} \times 100\,\%$$

Wherein:

At --- Peak area of the unknown impurity in the test solution;

Ar --- Average peak area of the main peak in the reference solution;

Cr --- Concentration of the reference solution (mg/mL);

Ct --- Concentration of the test solution (mg/mL);

P --- Concentration of the TY-9591 reference standard

The concentration of impurity B is calculated using the above formula.

**Example 1 Tablet 1 and its Preparation (Formulation (40 mg))**

| Name of raw and auxiliary materials | Percentage by Weight (w/w) | mg/ Tablet | Function |
|---|---|---|---|
| TY-9591 (D90= 4.8 μm) | 19.04 | 47.60 | Pharmaceutical substance |
| Mannitol | 58.96 | 147.40 | diluent |
| Microcrystalline cellulose | 15.00 | 37.50 | diluent |
| Low-substituted hydroxypropyl cellulose | 5.00 | 12.50 | Disintegrant |
| Sodium stearoyl fumarate (internal) | 0.50 | 1.25 | lubricant |
| Sodium stearoyl fumarate (external) | 1.50 | 3.75 | lubricant |

(continued)

| Name of raw and auxiliary materials | Percentage by Weight (w/w) | mg/ Tablet | Function |
|---|---|---|---|
| Total | 100 | 250 | |
| Note: The TY-9591 used contains one molecule of methanesulfonic acid; 47.6 mg of the salt form is equivalent to 40 mg of free TY-9591 base. | | | |

TY-9591 was subjected to micronization to control its D90 particle size within 20 μm (actual D90 was 4.8 μm). Tablet 1 was manufactured using a dry mixing/compaction process with the materials listed in the table above. TY-9591, mannitol, microcrystalline cellulose, and low-substituted hydroxypropyl cellulose were sequentially added to a 5 L mixing hopper, the mixing rotation speed was set to 10 rpm, and mixing was continued for 15 minutes. Then Sodium stearoyl fumarate (internal) was added to the 5 L mixing hopper, the mixing speed was set to 10 rpm, and mixing was continued for 5 minutes to obtain a premix. The premix was added to the dry granulator for dry granulation, with a roller gap of 1.5 - 2.5 mm (specifically 1.9 - 2.0 mm), a roller speed of 7.0 - 11.0 rpm (specifically 9 rpm), and a hydraulic pressure of 35 - 45 bar (specifically 45 bar), to obtain dry granules. The granules collected after dry granulation and the calculated amount of sodium stearoyl fumarate (external) were added to a 5 L mixing hopper, the rotation speed was set to 10 rpm and the mixing time to 5 minutes (i.e., total mixing time). This mixture is compressed into tablets by using a punch to obtain plain tablets 1 (hardness 98.8 - 109.8 N).

A 20% (w/w) coating suspension was prepared from a film-coating premix (7.5 mg/tablet, whose main components were polyvinyl alcohol, titanium dioxide, polyethylene glycol, talc, and red iron oxide), and the plain tablets 1 were coated with the coating suspension by a 3.8 % weigh gain to the weight of the plain tablets to form coated tablets 1 weighing 262.5 mg.

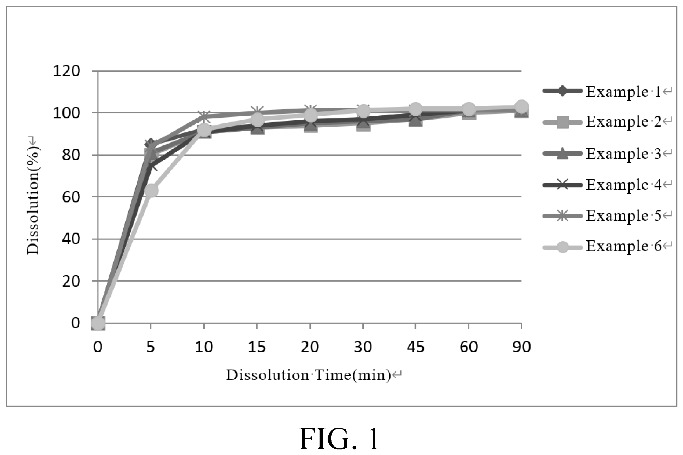

The dissolution results for Tablet 1 (the dissolution medium was a pH 1.2 sodium chloride-hydrochloric acid buffer solution containing 0.2% NaCl +7 mL of hydrochloric acid) were shown in Table 1 and Figure 1. Under these dissolution conditions, Tablet 1 dissolved completely, with 95% of the labeled amount dissolved by 45 minutes, meeting the standard requirements.

**Example 2~6 Tablet 2~6**

The same as Example 1, except that the particle size of TY-9591 differs, as shown in the table below.

| Example | particle size of TY-9591(D90) |
|---|---|
| Example 2 | 16 μm |
| Example 3 | 95 μm |
| Example 4 | 195 μm |
| Example 5 | 246 μm |
| Example 6 | 494 μm |

The dissolution results for tablets 2 - 6 (the dissolution medium was a pH 1.2 sodium chloride-hydrochloric acid buffer solution containing 0.2% NaCl + 7 mL of hydrochloric acid) were shown in Table 1 and Figure 1.

Table 1

| Example | Dissolution time (min) | 5 | 10 | 15 | 20 | 30 | 45 | 60 | 90 |
|---|---|---|---|---|---|---|---|---|---|
| Example 1 | Average dissolution rate % | 85 | 92 | 94 | 95 | 97 | 99 | 101 | 102 |
| | RSD% | 15 | 5 | 4 | 3 | 2 | 1 | 2 | 2 |
| Example 2 | Average dissolution rate % | 80 | 91 | 93 | 94 | 95 | 97 | 100 | 101 |
| | RSD% | 11 | 3 | 2 | 4 | 2 | 3 | 2 | 2 |
| Example 3 | Average dissolution rate % | 81 | 91 | 93 | 95 | 96 | 97 | 101 | 101 |
| | RSD% | 12 | 5 | 4 | 3 | 2 | 2 | 3 | 2 |

(continued)

| Example | Dissolution time (min) | 5 | 10 | 15 | 20 | 30 | 45 | 60 | 90 |
|---|---|---|---|---|---|---|---|---|---|
| Example 4 | Average dissolution rate % | 75 | 91 | 94 | 96 | 97 | 99 | 101 | 101 |
| | RSD% | 15 | 7 | 4 | 4 | 3 | 2 | 3 | 2 |
| Example 5 | Average dissolution rate % | 84 | 98 | 100 | 101 | 101 | 101 | 101 | 101 |
| | RSD% | 13 | 2 | 2 | 2 | 2 | 2 | 2 | 2 |
| Example 6 | Average dissolution rate % | 63 | 92 | 97 | 99 | 101 | 102 | 102 | 103 |
| | RSD% | 23 | 5 | 4 | 3 | 2 | 2 | 2 | 2 |

As shown in Table 1, this formulation was suitable for a particle size range of D90 20 - 280 μm; in particular, dissolution met the standard for both small particle TY-9591 (D90 < 20 μm) and large particle TY-9591 (D90 > 300 μm). The advantage of this formulation lied in its ability to ensure complete release of micronized drugs (D90 < 5 μm) despite their large specific surface area and susceptibility to decomposition and deliquescence. In contrast, the formulation reported in Patent CN 110013468 B failed to achieve complete drug dissolution when D90 is < 20 μm (Comparative Example 1(a)). In the large particle size range (Example 6, D90 = 494 μm > 280 μm), the drug was also able to dissolve completely due to the promoting effect of the lubricant applied both internally and externally on drug dissolution. Furthermore, tablets prepared using the formulation of the present invention (e.g., Examples 1 - 6) are also capable of complete dissolution in a dissolution medium of phosphate buffer at pH 6.8. Therefore, compared to the solution reported in Patent CN 110013468 B, the present invention exhibits significant advantages in both the small and large particle size ranges.

**Example 7~10**

To investigate the effects of internal and external lubricant addition on tablets, Examples 7~10 examined the ratios of internal and external lubricant addition. The API used had a particle size of D90 = 222 μm. The specific ratios were shown in the table below (the dissolution medium was a pH 1.2 sodium chloride-hydrochloric acid buffer solution consisting 0.2% NaCl + 7 mL hydrochloric acid).

| Name of raw and auxiliary materials | Example 7 | Example 8 | Example 9 | Example 10 |
|---|---|---|---|---|
| | Percentage by Weight (w/w) | Percentage by Weight (w/w) | Percentage by Weight (w/w) | Percentage by Weight (w/w) |
| TY-9591 | 19.04 | 19.04 | 19.04 | 19.04 |
| Mannitol | 58.96 | 58.96 | 58.96 | 58.96 |
| Microcrystalline cellulose | 15.00 | 15.00 | 15.00 | 15.00 |
| Low-substituted hydroxypropyl cellulose | 5.00 | 5.00 | 5.00 | 5.00 |
| Sodium stearoyl fumarate (internal) | 1.00 | 0.50 | 0.33 | 0 |
| Sodium stearoyl fumarate (external) | 1.00 | 1.50 | 1.67 | 2.00 |

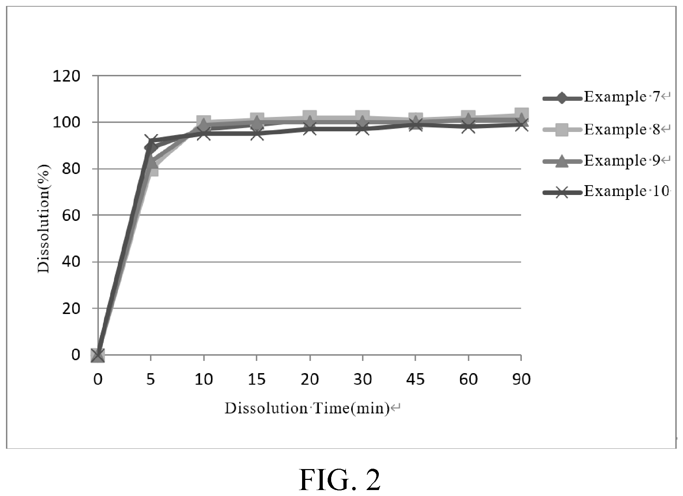

As shown in Table 2 and Figure 2, under the conditions of this formulation (D90 = 222 μm), the internal or external of lubricant had no significant effect on drug dissolution within this particle size range. However, considering the need for drug dissolution in the larger particle size range, the method of internal and external (1:3) is preferred. In this method, the lubricant dosage is 2%, whereas the formulation reported in Patent CN 110013468 B used a lubricant dosage of 1%. Therefore, the powder prepared using this method exhibits better flowability, which is more conducive to the scale-up of the formulation.

Table 2

| Example | Dissolution time (min) | 5 | 10 | 15 | 20 | 30 | 45 | 60 | 90 |
|---|---|---|---|---|---|---|---|---|---|
| Example 7 | Average dissolution rate % | 89 | 97 | 99 | 101 | 100 | 101 | 101 | 102 |
| | RSD% | 8 | 4 | 3 | 3 | 2 | 2 | 2 | 1 |

(continued)

| Example | Dissolution time (min) | 5 | 10 | 15 | 20 | 30 | 45 | 60 | 90 |
|---|---|---|---|---|---|---|---|---|---|
| Example 8 | Average dissolution rate % | 80 | 100 | 101 | 102 | 102 | 101 | 102 | 103 |
| | RSD% | 3 | 4 | 4 | 3 | 2 | 1 | 2 | 2 |
| Example 9 | Average dissolution rate % | 83 | 99 | 100 | 100 | 100 | 100 | 101 | 101 |
| | RSD% | 14 | 3 | 3 | 2 | 3 | 2 | 1 | 2 |
| Example 10 | Average dissolution rate % | 92 | 95 | 95 | 97 | 97 | 99 | 98 | 99 |
| | RSD% | 1 | 1 | 1 | 0 | 0 | 1 | 1 | 1 |

**Example 11, 12, 13 Tablets 11, 12, 13**

As in Example 3, the difference lies in the coating weight gain; the particle size of the TY-9591 is D90=112 μm, as shown in the table below.

| | Coating weight ratio (%) |
|---|---|
| Example 11 | 2.1 |
| Example 12 | 3.8 |
| Example 13 | 5.1 |

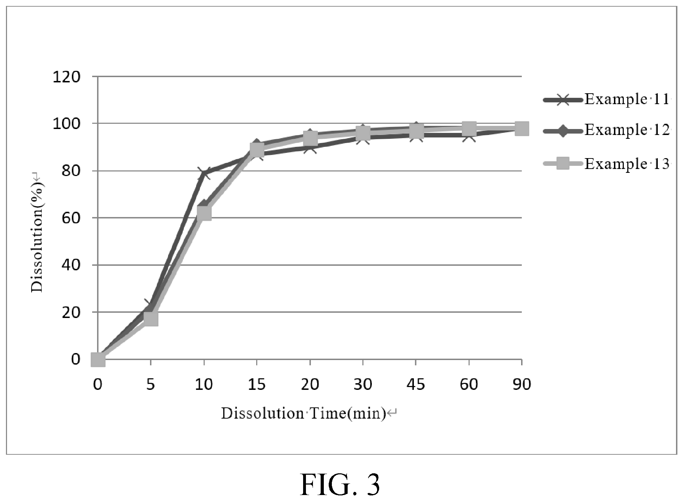

The dissolution results for tablets 11, 12, and 13 (the dissolution medium was a pH 1.2 sodium chloride-hydrochloric acid buffer solution consisting 0.2% NaCl + 7 mL hydrochloric acid) are shown in Table 3 and Figure 3.

Table 3

| Example / Dissolution rate (%) / Time(min) | 0 | 5 | 10 | 15 | 20 | 30 | 45 | 60 | 90 |
|---|---|---|---|---|---|---|---|---|---|
| Example 11 | 0 | 23 | 79 | 87 | 90 | 94 | 95 | 95 | 98 |
| Example 12 | 0 | 21 | 65 | 91 | 95 | 97 | 98 | 98 | 98 |
| Example 13 | 0 | 17 | 62 | 89 | 94 | 96 | 97 | 98 | 98 |

As shown in Table 3 and Figure 3, the dissolution curves of the coated tablets with different coating weight increases are essentially consistent and meet the standards.

**Examples 14 and 15 Tablets 14 and 15**

As in Example 3, except that the hardness of plain tablets 14 and 15 differs, as shown in the table below.

| | Hardness of plain tablets (N) |
|---|---|
| Example 3 | 100 |
| Example 14 | 70 |
| Example 15 | 130 |

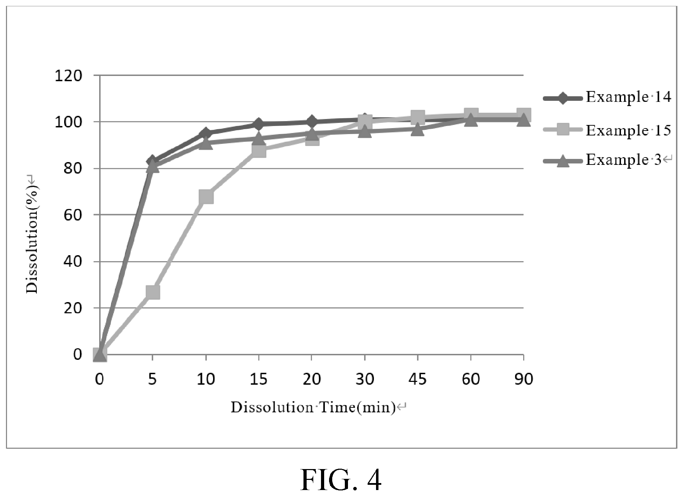

The dissolution results for tablets 14 and 15 (the dissolution medium was a pH 1.2 sodium chloride-hydrochloric acid buffer solution consisting 0.2% NaCl + 7 mL hydrochloric acid) were shown in Table 4 and Figure 4.

Table 4

| Example \ Dissolution rate (%) \ Time(min) | 0 | 5 | 10 | 15 | 20 | 30 | 45 | 60 | 90 |
|---|---|---|---|---|---|---|---|---|---|
| Example 14 | 0 | 83 | 95 | 99 | 100 | 101 | 101 | 101 | 101 |
| Example 15 | 0 | 27 | 68 | 88 | 93 | 100 | 102 | 103 | 103 |

As shown in Table 4 and Figure 4, there were certain differences in the dissolution curves of plain tablets with varying hardness, primarily in the dissolution rates between 5 and 15 minutes. As tablet hardness increased, the dissolution rate slowed down; however, the drug was essentially fully released by 30 minutes, which complied with the standard.

**Examples 16-19 Tablets 16-19**

As in Example 3, the differences lied in the feed speed, roller gap, roller speed, and hydraulic pressure for dry granulation. The particle size of the TY-9591 was D90 = 112 μm, as shown in Table 5 below.

Table 5

| | Feed speed (rpm) | Roller gap (mm) | Roller speed (rpm) | Hydraulic pressure (bar) |
|---|---|---|---|---|
| Example 16 | 58.2 | 2.5 | 7 | 35 |
| Example 17 | 61.3 | 1.5 | 11 | 35 |
| Example 18 | 81.0 | 2.5 | 11 | 55 |
| Example 19 | 43.0 | 1.5 | 7 | 55 |

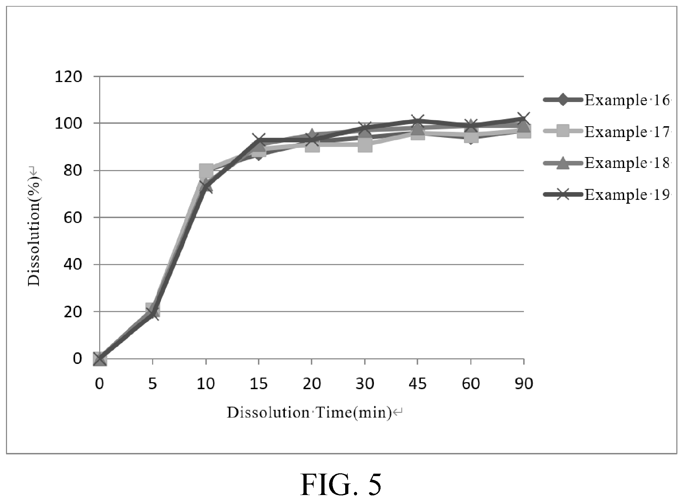

The dissolution results for tablets 16 - 19 (the dissolution medium was a pH 1.2 sodium chloride-hydrochloric acid buffer solution consisting 0.2% NaCl + 7 mL hydrochloric acid) were shown in Table 6 and Figure 5.

Table 6

| Example \ Dissolution rate (%) \ Time(min) | 5 | 10 | 15 | 20 | 30 | 45 | 60 | 90 |
|---|---|---|---|---|---|---|---|---|
| Example 16 | 20 | 80 | 87 | 92 | 94 | 96 | 94 | 97 |
| Example 17 | 21 | 80 | 89 | 91 | 91 | 96 | 95 | 97 |
| Example 18 | 21 | 74 | 91 | 95 | 97 | 98 | 99 | 99 |
| Example 19 | 19 | 73 | 93 | 93 | 98 | 101 | 99 | 102 |

As shown in Table 5 and Figure 5, changing the feed rate, roller gap, roller speed, and hydraulic pressure during dry granulation yielded similar dissolution results, with dissolution rates exceeding 80% after 15 minutes, which complied with the standard.

**Example 20 Tablet 20**

As in Example 3, the difference lied in the varying amounts of each component, as shown in the table below.

| Name of raw and auxiliary materials | Percentage by Weight (w/w) | mg/Tablet | Function |
|---|---|---|---|
| TY-9591 | 19.04 | 47.60 | Pharmaceutical substance |
| Mannitol | **53.96** | 134.90 | diluent |
| Microcrystalline cellulose | **20.00** | 50.00 | diluent |
| Low-substituted hydroxypropyl cellulose | 5.00 | 12.50 | Disintegrant |
| Sodium stearoyl fumarate (internal) | 0.50 | 1.25 | lubricant |
| Sodium stearoyl fumarate (external) | 1.50 | 3.75 | lubricant |
| Total | 100 | 250 | |

Given that microcrystalline cellulose possesses strong binding properties and good compressibility-and is also known as a "dry binder"-increasing its proportion helps improve tablet compressibility. Therefore, Tablet 20 was formulated by increasing the proportion of microcrystalline cellulose by 5% compared to Tablet 3. The summary of tableting data for Examples 3 and 20 was shown in Table 7 below.

Table 7

| No. | Tablet Weight (mg) | Hardness (N) | Tablet Thicknes s (mm) | Disintegratio n Time | Main Compressio n Thickness (mm) |
|---|---|---|---|---|---|
| Example 3 | 252.5 | 82.3 | 4.00 | 2’06" | 3.1 |
| | 253.0 | 119.6 | 3.88 | / | 2.8 |
| Example 20 | 252.5 | 81.4 | 4.00 | 1’27" | 3.1 |
| | 252.4 | 139.3 | 3.82 | 1’52" | 2.7 |

As shown in the table above, for the same tablet thickness, the hardness of tablets produced from the two formulations was similar (around 80 N), and the main compression thickness is consistent, this indicated that the compressibility of the two formulations was comparable. Tablet 20 achieved a maximum hardness of approximately 140 N, indicating some improvement in compressibility; however, due to the increased proportion of microcrystalline cellulose, the flowability of the powder mixture was slightly poorer than that of Tablet 3.

**Example 21 Tablet 21 and its preparation (Formulation (10 mg))**

| Name of raw and auxiliary materials | Percentage by Weight (w/w) | mg/Tablet | Function |
|---|---|---|---|
| TY-9591 | 19.04 | 11.9 | Pharmaceutical substance |
| Mannitol | 58.96 | 36.85 | diluent |
| Microcrystalline cellulose | 15.00 | 9.375 | diluent |
| Low-substituted hydroxypropyl cellulose | 5.00 | 3.125 | Disintegrant |
| Sodium stearoyl fumarate (internal) | 0.50 | 0.3125 | lubricant |
| Sodium stearoyl fumarate (external) | 1.50 | 0.9375 | lubricant |
| Total | 100 | 62.5 | |
| Note: The used TY-9591 contained one molecule of methanesulfonic acid; 11.9 mg of the salt form was equivalent to 10 mg of free TY-9591 base. | | | |

Tablet 21 was prepared using a dry mixing/compaction process with the materials listed in the table above. TY-9591 methanesulfonate salt, mannitol, microcrystalline cellulose, and low-substituted hydroxypropyl cellulose were added sequentially to a 5-L mixing hopper, the mixing speed was set to 10 rpm, and mixing was continued for 15 minutes. Then sodium stearoyl fumarate (internal ingredient) to the 5 L mixing hopper, the mixing speed was set to 10 rpm, and mixed for 5 min. The premix was added to the dry granulator for dry granulation, with a roller gap of 1.5 - 2.5 mm, a roller speed of 7.0 - 11.0 rpm, and a hydraulic pressure of 35 - 45 bar. The granules collected after dry granulation and the calculated amount of sodium stearoyl fumarate (external) were added to a 5 L mixing hopper, the mixing speed was set to 10 rpm and mixed for 5 minutes. This mixture was compressed into tablets by using a punch to obtain plain tablets 21 (hardness 98.8 - 109.8 N).

A 20% (w/w) coating suspension was prepared from the film-coating premix, and and the plain tablets were coated with the coating suspension by a 3 % weigh gain to the weight of the plain tablets to form coated tablets 21 weighing 64.4 mg.

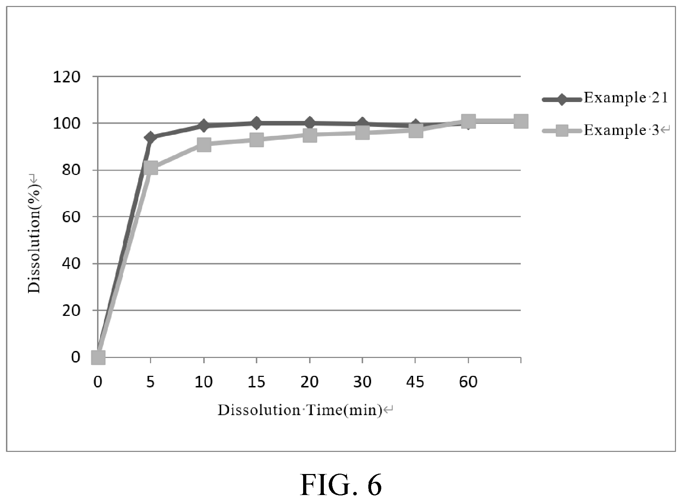

The dissolution curve of Tablet 21 in a sodium chloride hydrochloride dissolution medium at pH 1.3 was shown in Figure 6. The dissolution results were similar to those of the 40 mg strength tablet, with dissolution exceeding 80% within 15 minutes, complying with the standard.

**Example 22** The inventor conducted a scale-up production according to the formulation of Example 1 to obtain tablets 22. In this batch, TY-9591 was not subjected to micronization, and its particle size was D90=145 μm. Except for the use of a 30 L mixing hopper and a coating weight gain of 3.2%, the preparation process parameters, such as mixing speed and mixing time, were the same as those in Example 1. The inventors collected data on relevant substances, content, and dissolution under long-term conditions (25°C ± 2°C/60% RH ± 5% RH) and accelerated conditions (40°C ± 2°C/75% RH ± 5% RH), as shown in Tables 7 and 8 below. Based on the data from the 3-year long-term storage and 6-month accelerated storage, it was concluded that Tablet 22 prepared according to the formulation of Example 1 exhibited good storage stability.

Table 8 summarized the results of the long-term stability testing for Tablet 22 (40 mg strength). The dissolution medium was a pH 1.2 sodium chloride-hydrochloric acid buffer solution consisting 0.2% NaCl + 4 mL hydrochloric acid.

Table 8

| Test Conditions | | 25±2°C, 60%±5%RH | | | | | | | |
|---|---|---|---|---|---|---|---|---|---|
| **Project Overview** | | **0 mont h** | **3 mont h** | **6 mont h** | **9 mont h** | **12 mont h** | **18 mont h** | **24 mont h** | **36 mont h** |
| Concentration | | 99.3 % | 99.4 % | 99.6 % | 98.2 % | 97.3 % | 98.2 % | 98.6 % | 99.2 % |
| Releva nt substan ces | Impurit y B | 0.11 % | 0.15 % | 0.16 % | 0.13 % | 0.13 % | 0.09 % | <RL (RL= 0.05 %) | <RL (RL= 0.05 %) |
| | Single Unkno wn Impurit y | RRT =1.07 8 0.05 % RRT =1.22 6 0.05 % RRT =1.25 7 0.09 % | RRT =1.082 0.07 % RRT=1.262 0.07 % | RRT =1.081 0.07 % RRT=1.263 0.07 % | RRT =1.081 0.07 % RRT=1.254 0.07 % | RRT =1.079 0.07 % RRT=1.248 0.07 % | RRT =1.08 1 0.08 % RRT =1.23 7 0.05 % RRT =1.25 3 0.07 % | RRT =1.085 0.09 % RRT=1.267 0.07 % | RRT =1.08 3 0.10 % RRT =1.24 0 0.06 % RRT =1.25 7 0.08 % |
| | Total impuriti es | 0.31 % | 0.29 % | 0.29 % | 0.27 % | 0.26 % | 0.29 % | 0.16 % | 0.24 % |
| Dissolution rate (mean value, dissolution time: 30 min) | | 97% | 98% | 98% | 98% | 95% | 96% | 98% | 97% |

As shown in Table 8, all major parameters in the long-term stability study fluctuated within acceptable limits, with no significant differences observed across time points, indicating good stability. Specifically,

1) Regarding concentration, the test results fluctuated within the normal range.
2) Regarding relevant substances, although the test results for impurity B, the single unknown impurity, and total impurities showed some fluctuation, the range of variation was small, and no significant differences were observed across time points.
3) Regarding dissolution, the test results fluctuated within the normal range and complied with the Chinese Pharmacopoeia.

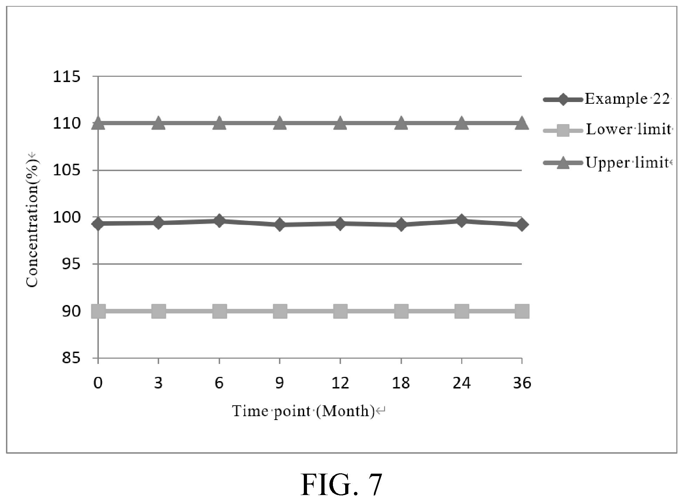

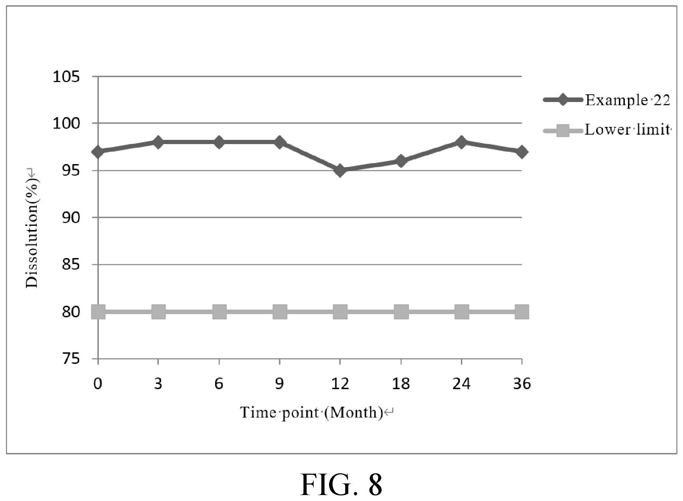

Figures 7 and 8 showed the content and dissolution curves of the tablet 22 prepared according to the formulation of Example 1 during long-term stability testing.
As shown in Figure 7, although there were some fluctuations in concentration at various time points during the long-term stability testing, all values remained within acceptable standard range.
As shown in Figure 8, although there were some fluctuations in dissolution at various time points during the long-term stability testing, all values remained within acceptable standard range and complied with the Chinese Pharmacopoeia.
Table 9 summarized the results of the accelerated stability testing for Tablet 22 (40 mg strength), using a dissolution medium of sodium chloride-buffered hydrochloric acid at pH 1.3.

Table 9

| **Test Conditions** | | **40±2**°C, **75%±5%RH** | | | | |
|---|---|---|---|---|---|---|
| **Project Overview** | | **0 month** | **1 month** | **2 month** | **3 month** | **6 month** |
| Concentration | | 99.3% | 98.4% | 99.0% | 97.9% | 96.4% |
| Releva nt substan ces | Impurit y B | 0.11% | 0.10% | 0.08% | 0.09% | 0.09% |
| | Single Unkno wn Impurit y | RRT=1.07 8 0.05% RRT=1.22 6 0.05% RRT=1.25 7 0.09% | RRT=1.08 2 0.07% RRT=1.26 8 0.07% | RRT=1.08 6 0.08% RRT=1.28 6 0.08% | RRT=1.08 1 0.09% RRT=1.26 1 0.07% | RRT=1.08 1 0.12% RRT=1.20 3 0.05% RRT=1.26 3 0.07% |
| | Total impuriti es | 0.31% | 0.24% | 0.24% | 0.25% | 0.33% |
| Dissolution rate (mean value, dissolution time: 30 min) | | 97% | 97% | 98% | 96% | 97% |

As shown in Table 9, all key indicators of acceleration stability fluctuated within a certain range, with no significant differences in the data across different time points, indicating good stability. Specifically,

1) Regarding concentrations, the test results fluctuated within the normal range.
2) Regarding relevant substances, although the test results for impurity B, the single unknown impurity, and total impurities showed some fluctuation, the range of variation was small, and there were no significant differences across the various time points.
3) Regarding dissolution, the test results fluctuated within the normal range and complied with the Chinese Pharmacopoeia.

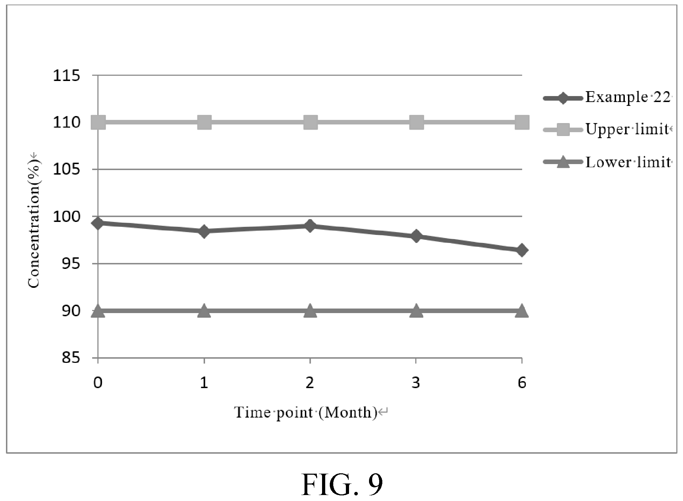

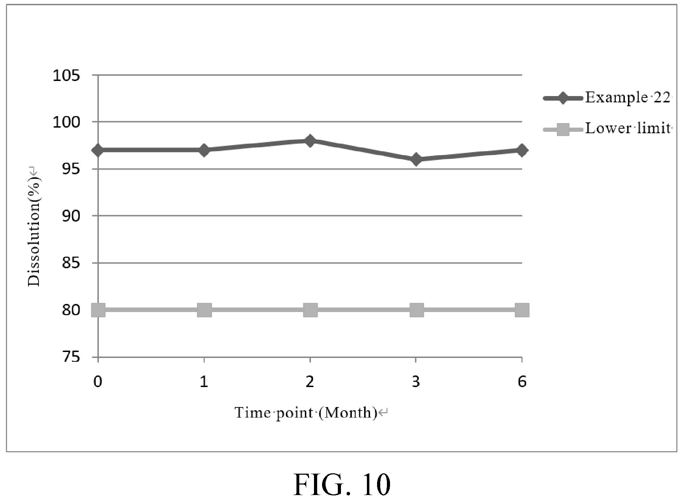

Figures 9 and 10 showed the content and dissolution curves obtained from accelerated stability testing of the tablet 22 prepared according to the formulation of Example 1.
As shown in Figure 9, although there were some fluctuations in the concentration at various time points during the accelerated stability testing, all results remained within acceptable standard range.
As shown in Figure 10, although there were some fluctuations in the dissolution rate at various time points during the long-term stability testing, all results remained within acceptable standard range and complied with the Chinese Pharmacopoeia.
The stability of the micronized sample (Example 1) and the 10 mg strength sample also complied with the standards.

**Example 23**

In-vivo exposure levels of Tablet 22

A single-dose study was conducted in healthy volunteers, and the pharmacokinetic data were shown in Table 10. The results indicated that a single oral dose of 80 mg of Tablet 22 (equivalent to of TY-9591 free base, 40 mg*2 tablets) resulted in good systemic exposure. Furthermore, the pharmacokinetic parameters were comparable in subjects administered the drug under both fasting and high-fat meal conditions, indicating that drug administration was not affected by food intake; the drug might be taken either on an empty stomach or after a meal.

Table 10

| | **TY-9591** | | | | | |
|---|---|---|---|---|---|---|
| Group | $T_{max}$ | $C_{max}$ | $AUC_{0-72h}$ | $AUC_{0-t}$ | $AUC_{0-\infty}$ | $t_{1/2}$ |
| | h | ng/mL | h*ng/mL | h*ng/mL | h*ng/mL | h |
| TY-9591 (Fasting) | 5.99 | 67.83 | 2334 | 3884 | 3927 | 51.25 |
| TY-9591 (After high-fat meal) | 5.95 | 69.02 | 2672 | 4305 | 4349 | 48.60 |

**Comparative Example 1**

Same as Example 3, except that no coating is applied.

Table 11

| Dissolution time | 5 min | 10 min | 15 min | 20 min | 30 min | 45 min | 60 min | 90 min |
|---|---|---|---|---|---|---|---|---|
| Uncoated tablet | 88 | 95 | 99 | 100 | 101 | 101 | 101 | 101 |

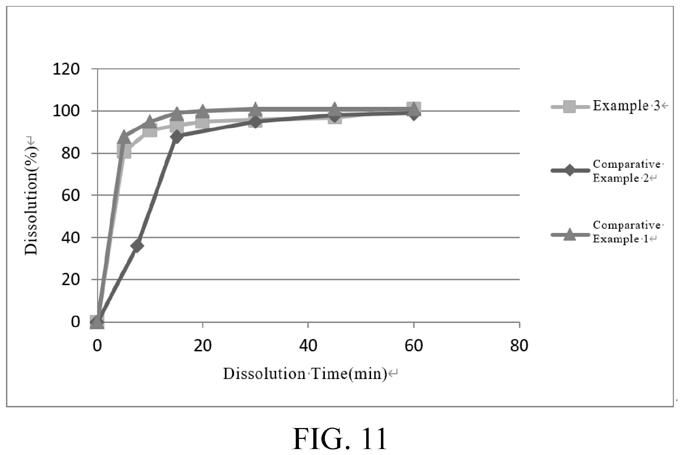

As shown in Table 11 and Figure 11, the dissolution rate of the plain tablet was slightly faster than that of coated tablet 3, but the difference was not statistically significant. However, as shown in Table 12, after coating, the level of Impurity B in the plain tablet increased slightly after 20 days of storage at room temperature, while the level of Impurity B in the coated tablet remained essentially unchanged, indicating that coating improves its stability.

Table 12

| Process Name/Sample | Impurity B/% | |
|---|---|---|
| | 0 day | 20 days (Laboratory placement) |
| API | N/A | 0.13 |
| Premix | 0.09 | 0.09 |
| Dry granulation | 0.09 | 0.14 |
| Uncoated tablets | 0.12 | 0.19 |
| Coated tablets | 0.15 | 0.16 |

Table 13 summarized the hardness - disintegration times of the tablets obtained in Example 3 and Comparative Example 1.

Table 13

| Example 3 | | Comparative Example 1 | |
|---|---|---|---|
| Hardness(N) | Disintegration time | Hardness(N) | Disintegration time |
| 42.2 | 1’24’’ | 45.1 | 1’57’’ |
| 55.9 | 2’08’’ | 60.8 | 2’10’’ |
| 67.7 | 2’27’’ | 67.7 | 2’49’’ |

(continued)

| Example 3 | | Comparative Example 1 | |
|---|---|---|---|
| Hardness(N) | Disintegration time | Hardness(N) | Disintegration time |
| 82.4 | 2’06’’ | 77.5 | 3’24’’ |
| 108.9 | 2’20’’ | 95.1 | 5’29’’ |

As shown in Table 13, the disintegration time of Example 3 exhibited no significant variation with hardness, whereas the disintegration time of Comparative Example 1 increased significantly as hardness increased.

**Comparative Example 2**

Same as Example 1, except that the preparation process followed the parameters specified in Example 9 of Patent CN105848647A. Specifically:
AZD9291 methanesulfonate salt, mannitol, microcrystalline cellulose, and low-substituted hydroxypropyl cellulose were sequentially added to a 25-L mixing hopper. The mixing speed was set to 15 rpm, and mixing was conducted for 58 minutes. Then Sodium stearoyl fumarate (0.5%) was added to the mixing hopper, the mixing speed was set to 15 rpm, and mixing was conducted for 9.5 minutes. The premix was fed into a dry granulator for dry granulation, with a roller gap of 2 mm, a drum speed of 10.1 - 10.2 rpm (25 mm drum), and a screw speed of 22.4 - 22.9 rpm. Ground the resulting ribbon using a Comil U3 with a granulator speed of 100 rpm and a screen aperture size of 1.27 mm. The granules collected after granulation and the calculated amount of sodium stearoyl fumarate (external) was added to the mixing hopper, the speed was set to 15 rpm, and mixed for 5 minutes. The tablet cores were compressed using a tablet press equipped with a 9 mm punch (40 mg strength).

A 20% (w/w) coating suspension was prepared from the film-coating premix, and the plain tablets were coated with the coating suspension by a 5% weigh gain to the weight of the plain tablets to form coated tablets weighing 262.5 mg.

Figure 11 showed a comparison of the dissolution curves of Example 9 of Patent CN105848647A in a sodium chloride buffer solution at pH 1.3 with the dissolution curve of Example 3 of the present invention. The dissolution rate of Example 3 of the present invention at 5 minutes was significantly higher than that of the comparative example at 7.5 minutes. The optimized preparation process of the present invention offered the significant advantage of a faster dissolution rate, while also substantially shortening the preparation time and reducing production costs.

All literatures mentioned in the present invention are incorporated by reference herein, as though individually incorporated by reference. Additionally, it should be understood that after reading the above teaching, many variations and modifications may be made by the skilled in the art, and these equivalents also fall within the scope as defined by the appended claims.

## Claims

**1.** A pharmaceutical composition, **characterized in that** the pharmaceutical composition is a tablet comprising a tablet core and a coating located on the outer surface of the tablet core;

the tablet core comprises the following components:

| | Part by weight | Function |
|---|---|---|
| Methanesulfonate salt of Compound of Formula I | 18.5 - 19.5 | Active ingredient |
| Mannitol | 57 - 60 | Primary diluent |
| Microcrystalline cellulose | 12 - 17 | Secondary diluent |
| Low-substituted hydroxypropyl cellulose | 3-7 | Disintegrant |
| Sodium stearoyl fumarate (internal) | 0.3-0.7 | Primary lubricant |
| Sodium stearoyl fumarate (external) | 1.3 - 1.7 | Secondary lubricant |

the coating completely covers the tablet core, and the mass of the coating is 1 - 6% of the mass of the tablet core;

Formula I

the pharmaceutical composition was prepared as follows:

1) providing the methanesulfonate salt of the compound of Formula I, mannitol, microcrystalline cellulose, low-substituted hydroxypropyl cellulose, sodium stearoyl fumarate, and a coating suspension;
2) mixing the methanesulfonate salt of the compound of Formula I, mannitol, microcrystalline cellulose, and low-substituted hydroxypropyl cellulose to obtain a first mixture;
3) mixing the sodium stearoyl fumarate for internal incorporation with the first mixture to obtain a second mixture;
4) subjecting the second mixture to dry granulation to obtain a first granules;
5) mixing the first granules with sodium stearoyl fumarate for external incorporation to obtain a third mixture;
6) pressing the third mixture to obtain a plain tablet;
7) coating the plain tablet with the coating suspension to obtain the pharmaceutical composition.

**2.** The pharmaceutical composition according to claim 1, **characterized in that** the D90 of the active ingredient is 1 - 500 μm.

**3.** The pharmaceutical composition according to claim 1, **characterized in that** the methanesulfonate salt of the compound of formula I is the mono-methanesulfonate crystal form of the compound of formula I.

**4.** The pharmaceutical composition according to claim 3, **characterized in that** the X-ray powder diffraction patterns of the crystal form having diffraction peaks at the 2θ angle (where the unit of the 2θ angle is °): 7.1±0.2, 8.5±0.2, 9.4 ± 0.2, 10.3 ± 0.2, 12.6 ± 0.2, 14.4 ± 0.2, 15.1 ± 0.2, 15.6 ± 0.2, 16.3 ± 0.2, 17.0 ± 0.2, 17.3 ± 0.2, 17.7 ± 0.2, 18.2±0.2, 18.7±0.2, 19.4±0.2, 19.7±0.2, 20.2±0.2, 20.7±0.2, 21.6±0.2, 22.0±0.2, 22.8±0.2, 23.5±0.2, 24.2±0.2, 24.8 ±0.2, 25.6±0.2, 26.0±0.2, 26.9±0.2, 27.7±0.2, 28.2±0.2, 29.5±0.2, 30.7±0.2, 31.7 ± 0.2, 32.5 ± 0.2, 33.1 ± 0.2, 33.8 ± 0.2, 34.6 ± 0.2, 34.9 ± 0.2, 35.6 ± 0.2, 37.9 ± 0.2, and 38.7 ± 0.2.

**5.** The pharmaceutical composition according to claim 1, **characterized in that** the pharmaceutical composition has one or more of the following features:

1) in a sodium chloride buffer solution at pH 1.2, the dissolution rate of the active ingredient in the pharmaceutical composition is ≥50% at 10 minutes;
2) in a sodium chloride buffer solution at pH 1.2, the dissolution rate of the active ingredient in the pharmaceutical composition is ≥80% at 15 minutes;
3) in a sodium chloride buffer solution at pH 1.2, the dissolution rate of the active ingredient in the pharmaceutical composition is ≥85% at 20 minutes;
4) in a sodium chloride buffer solution at pH 1.2, the dissolution rate of the active ingredient in the pharmaceutical composition is ≥90% at 30 minutes;
5) in a sodium chloride buffer solution at pH 1.2, the dissolution rate of the active ingredient in the pharmaceutical composition is ≥96% at 45 minutes;
6) in a sodium chloride buffer solution at pH 1.2, the dissolution rate of the active ingredient in the pharmaceutical composition is ≥100% at 60 minutes;
7) after storage of the pharmaceutical composition for 3 years at 25±2°C and 60%±5% RH, the dissolution rate of the active ingredient in the pharmaceutical composition is ≥95% at 30 minutes in a sodium chloride buffer solution at pH 1.3;
8) after storage of the pharmaceutical composition for 6 months at 40±2°C and 75%±5% RH, the dissolution rate of the active ingredient in the pharmaceutical composition is ≥95% at 30 minutes in a sodium chloride buffer solution at pH 1.3.

**6.** A method for preparing the pharmaceutical composition according to claim 1, **characterized in that** it comprises steps of:

1) providing the methanesulfonate salt of the compound of Formula I, mannitol, microcrystalline cellulose, low-substituted hydroxypropyl cellulose, sodium stearoyl fumarate, and a coating suspension;
2) mixing the methanesulfonate salt of the compound of Formula I, mannitol, microcrystalline cellulose, and low-substituted hydroxypropyl cellulose to obtain a first mixture;
3) mixing the sodium stearoyl fumarate for internal incorporation with the first mixture to obtain a second mixture;
4) subjecting the second mixture to dry granulation to obtain a first granules;
5) mixing the first granules with sodium stearoyl fumarate for external incorporation to obtain a third mixture;
6) pressing the third mixture to obtain a plain tablet;
7) coating the plain tablet with the coating suspension to obtain the pharmaceutical composition.

**7.** The method according to claim 6, **characterized in that** the method comprises one or more of the following features:

1) In step 2), mixing speed of the mixing is 4 - 14 rpm;
2) In step 2), mixing time of the mixing is 10 - 20 min;
3) In step 3), mixing speed of the mixing is 4 - 14 rpm;
4) in step 3), mixing time of the mixing is 2 - 9 min;
5) in step 4), roller gap of the dry granulation is 1.5 - 2.5 mm;
6) in step 4), roller speed of the dry granulation is 7.0 - 10.0 rpm;
7) In step 4), hydraulic pressure of the dry granulation is 35 - 50 bar;
8) In step 5), mixing speed of the mixing is 4 - 14 rpm;
9) In step 5), mixing time of the mixing is 2 - 10 min.

**8.** The method according to claim 6, **characterized in that** the hardness of the plain tablet is 70 - 120 N.

**9.** A use of the pharmaceutical composition according to claim 1in the preparation of an antitumor drug.

**10.** The use according to claim 9, **characterized in that** the tumor is lung cancer.

120
100
80
60
40
20
0
Dissolution(%)
0
5
10
15
20
30
45
60
90
Dissolution Time(min)
Example 1
Example 2
Example 3
Example 4
Example 5
Example 6

FIG. 1

120
100
80
60
40
20
0
Dissolution(%)
0
5
10
15
20
30
45
60
90
Dissolution·Time(min)
Example·7
Example·8
Example·9
Example·10

FIG. 2

120
100
80
60
40
20
0
Dissolution(%)
0
5
10
15
20
30
45
60
90
Dissolution Time(min)
Example 11
Example 12
Example 13

FIG. 3

120
100
80
60
40
20
0
Dissolution(%)
0
5
10
15
20
30
45
60
90
Dissolution Time(min)
Example 14
Example 15
Example 3

FIG. 4

120
100
80
60
40
20
0
Dissolution(%)
0
5
10
15
20
30
45
60
90
Dissolution Time(min)
Example 16
Example 17
Example 18
Example 19

FIG. 5

120
100
80
60
40
20
0
Dissolution(%)
0
5
10
15
20
30
45
60
Dissolution Time(min)
Example 21
Example 3

FIG. 6

115
110
105
100
95
90
85
Concentration(%)
0
3
6
9
12
18
24
36
Time point (Month)
Example 22
Lower limit
Upper limit

FIG. 7

105
100
95
90
85
80
75
Dissolution(%)
0
3
6
9
12
18
24
36
Time point (Month)
Example 22
Lower limit

FIG. 8

115
110
105
100
95
90
85
Concentration(%)
0
1
2
3
6
Time point (Month)
Example 22
Upper limit
Lower limit

FIG. 9

105
100
95
90
85
80
75
Dissolution(%)
0
1
2
3
6
Time point (Month)
Example 22
Lower limit

FIG. 10

120
100
80
60
40
20
0
Dissolution(%)
0
20
40
60
80
Dissolution Time(min)
Example 3
Comparative Example 2
Comparative Example 1


FIG. 11

## INTERNATIONAL SEARCH REPORT

International application No.
**PCT/CN2024/129036**

**A. CLASSIFICATION OF SUBJECT MATTER**

A61K 9/32(2006.01)i; A61K 31/506(2006.01)i; A61K 9/20(2006.01)i; A61K 47/26(2006.01)i; A61K 47/38(2006.01)i; A61P 35/00(2006.01)i

According to International Patent Classification (IPC) or to both national classification and IPC

**B. FIELDS SEARCHED**

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

CNKI, CNABS, CNTXT, DWPI, SIPOABS, ENTXTC, STN, 百度, BAIDU: 氘代, AZD9291, 奥希替尼, 甘露醇, 微晶纤维素, 低取代羟丙基纤维素, 硬脂富马酸钠, 干法制粒, 润滑剂, 内加, 外加, 包衣, 稳定性, 晶型, deuterated, osimtinib, mannitol, hydroxypropyl cellulose, tablet, lubricant, sodium stearyl fumarate, 1638281-46-5

**C. DOCUMENTS CONSIDERED TO BE RELEVANT**

| Category* | Citation of document, with indication, where appropriate, of the relevant passages | Relevant to claim No. |
|---|---|---|
| Y | CN 110013468 A (BEIJING WINSUNNY PHARMACEUTICAL CO., LTD.) 16 July 2019 (2019-07-16)<br>claim 1, and description, paragraphs [0002]-[0003] and [0026], and embodiment 2 | 1-10 |
| Y | CN 105848647 A (ASTRAZENECA AB) 10 August 2016 (2016-08-10)<br>description, paragraphs [0435]-[0437] | 1-10 |
| Y | CN 108558835 A (TYK MEDICINES, INC.) 21 September 2018 (2018-09-21)<br>abstract, and claim 3 | 1-10 |
| A | CN 104140418 A (ZHU XIAOYUN) 12 November 2014 (2014-11-12)<br>claims 1-5 | 1-10 |
| A | CN 111362924 A (NANJING LEIZHENG PHARMACEUTICAL TECHNOLOGY CO., LTD.) 03 July 2020 (2020-07-03)<br>claims 5-6 and 10 | 1-10 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **19 January 2025** | **23 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/129036**

| Patent document cited in search report | Publication date (day/month/year) | Patent family member(s) | Publication date (day/month/year) |
|---|---|---|---|
| CN 110013468 A | 16 July 2019 | None | |
| CN 105848647 A | 10 August 2016 | CR 20160310 A | 23 September 2016 |
| | | AR 098989 A1 | 22 June 2016 |
| | | US 2022395502 A1 | 15 December 2022 |
| | | WO 2015101791 A1 | 09 July 2015 |
| | | US 2019111057 A1 | 18 April 2019 |
| | | CA 2933403 A1 | 09 July 2015 |
| | | CA 2933403 C | 29 March 2022 |
| | | PT 3089741 T | 11 June 2021 |
| | | DOP 2016000156 A | 15 August 2016 |
| | | IL 246186 A0 | 31 July 2016 |
| | | IL 246186 B | 30 July 2020 |
| | | ES 2873226 T3 | 03 November 2021 |
| | | UY 35933 A | 31 July 2015 |
| | | GB 201400034 D0 | 19 February 2014 |
| | | ZA 201605300 B | 26 October 2022 |
| | | US 2024189310 A1 | 13 June 2024 |
| | | SG 11201605339 QA | 28 July 2016 |
| | | US 2016324854 A1 | 10 November 2016 |
| | | US 10183020 B2 | 22 January 2019 |
| | | RS 61927 B1 | 30 June 2021 |
| | | TW 201609101 A | 16 March 2016 |
| | | TWI 702953 B | 01 September 2020 |
| | | SI 3089741 T1 | 31 August 2021 |
| | | CL 2016001609 A1 | 09 December 2016 |
| | | HRP 20210749 T1 | 25 June 2021 |
| | | PL 3089741 T3 | 27 September 2021 |
| | | GT 201600142 A | 12 August 2019 |
| | | DK 3089741 T3 | 07 June 2021 |
| | | HK 1225655 A1 | 15 September 2017 |
| | | US 2020360378 A1 | 19 November 2020 |
| | | KR 20160101720 A | 25 August 2016 |
| | | KR 102336378 B1 | 08 December 2021 |
| | | PE 20161170 A1 | 24 November 2016 |
| | | NZ 721298 A | 27 October 2017 |
| | | CY 1124848 T1 | 25 November 2022 |
| | | NI 201600098 A | 20 December 2016 |
| | | MY 183536 A | 25 February 2021 |
| | | EP 3089741 A1 | 09 November 2016 |
| | | EP 3089741 B1 | 10 March 2021 |
| | | PH 12016501310 A1 | 14 September 2016 |
| | | AU 2015204218 A1 | 07 July 2016 |
| | | AU 2015204218 B2 | 15 June 2017 |
| | | LT 3089741 T | 10 June 2021 |
| | | MX 2016008744 A | 08 September 2016 |
| | | MX 367358 B | 16 August 2019 |
| | | JP 2017501201 A | 12 January 2017 |
| | | JP 6588915 B2 | 09 October 2019 |
| | | HUE 054344 T2 | 30 August 2021 |
| | | EA 201691242 A1 | 30 December 2016 |

Form PCT/ISA/210 (patent family annex) (July 2022)

**INTERNATIONAL SEARCH REPORT**
**Information on patent family members**

International application No.
**PCT/CN2024/129036**

| Patent document cited in search report | | | Publication date (day/month/year) | Patent family member(s) | | | Publication date (day/month/year) |
|---|---|---|---|---|---|---|---|
| | | | | EA | 034243 | B1 | 21 January 2020 |
| | | | | EA | 034243 | B9 | 10 April 2020 |
| CN | 108558835 | A | 21 September 2018 | JP | 2020521003 | A | 16 July 2020 |
| | | | | JP | 6971390 | B2 | 24 November 2021 |
| | | | | EP | 3647312 | A1 | 06 May 2020 |
| | | | | EP | 3647312 | A4 | 10 March 2021 |
| | | | | EP | 3647312 | B1 | 10 May 2023 |
| | | | | ES | 2956943 | T3 | 05 January 2024 |
| | | | | US | 2020157080 | A1 | 21 May 2020 |
| | | | | US | 10882845 | B2 | 05 January 2021 |
| | | | | WO | 2018214886 | A1 | 29 November 2018 |
| CN | 104140418 | A | 12 November 2014 | CA | 2958095 | A1 | 18 February 2016 |
| | | | | CA | 2958095 | C | 26 September 2023 |
| | | | | US | 2018016258 | A1 | 18 January 2018 |
| | | | | US | 10414756 | B2 | 17 September 2019 |
| | | | | KR | 20170036107 | A | 31 March 2017 |
| | | | | KR | 101937704 | B1 | 14 January 2019 |
| | | | | AU | 2015303641 | A1 | 23 March 2017 |
| | | | | AU | 2015303641 | B2 | 12 April 2018 |
| | | | | JP | 2017523247 | A | 17 August 2017 |
| | | | | JP | 6418622 | B2 | 07 November 2018 |
| | | | | WO | 2016023422 | A1 | 18 February 2016 |
| | | | | EP | 3181559 | A4 | 21 June 2017 |
| | | | | EP | 3181559 | A1 | 03 October 2018 |
| | | | | EP | 3181559 | B1 | 14 November 2018 |
| CN | 111362924 | A | 03 July 2020 | | None | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 104140418 B
- CN 110950847 B
- CN 108558835 A
- CN 201280033773
- CN 201810017685
- CN 105848647 A
- CN 110013468 B


### Non-patent literature cited in the description


- **SAMBROOK et al.** Molecular Cloning: Laboratory Manual. Cold Spring Harbor Laboratory Press, 1989